Europäisches Patentamt

European Patent Office

Office européen des brevets

(19)

(11) Publication number: **0 051 962**
**B1**

(12) **EUROPEAN PATENT SPECIFICATION**

(45) Date of publication of patent specification: 24.08.88

(21) Application number: 81305148.9

(22) Date of filing: 29.10.81

(51) Int. Cl.⁴: **A 61 K 31/415**, A 61 K 33/04
// (A61K33/04, 31:415)

(54) Pharmaceutical composition comprising 4-carbamoyl-imidazolium-5-olate.

(30) Priority: 05.11.80 JP 156173/80
06.11.80 JP 156795/80

(43) Date of publication of application:
19.05.82 Bulletin 82/20

(45) Publication of the grant of the patent:
24.08.88 Bulletin 88/34

(84) Designated Contracting States:
BE CH DE FR GB IT LI NL SE

(56) References cited:
GB-A-1 397 732
US-A-4 181 731

JOURNAL OF THE AMERICAN CHEMICAL
SOCIETY, vol. 74, no. 11, June 1952, Easton, Pa,
US, Ch.S.MILLER "Substituted imidazoles as
precursors of the purines", pp. 2892-94

(73) Proprietor: SUMITOMO CHEMICAL COMPANY,
LIMITED
15 Kitahama 5-chome Higashi-ku
Osaka-shi Osaka 541 (JP)

(72) Inventor: Yamahira, Yoshiya
11-5, Makamicho 1-chome
Takatsuki (JP)
Inventor: Fujioka, Keiji
2-1, Kuwatacho
Ibaraki (JP)

(74) Representative: Geering, Keith Edwin et al
REDDIE & GROSE 16 Theobalds Road
London WC1X 8PL (GB)

Courier Press, Leamington Spa, England.

# 0 051 962

**Description**

The present invention relates to pharmaceutical preparations comprising, as active ingredient, 4-carbamoyl-imidazolium-5-olate or a salt or hydrate thereof (hereinafter referred to as "Compound A") and to a method for the production thereof.

Compound A can be prepared by the method described, for example, in J. Am. Chem. Soc. Vol. 74, 2892 (1952), and is useful as a therapeutic agent for the treatment of tumors, rheumatism and nephritis (U.S. Patent No. 4,181,731; French Patent No. 7727134; Canadian Patent No. 1,078,736).

It is also known that Compound A can be formulated into various dosage forms such as tablets, capsules, and injections.

It has now been found, however, that Compound A is so unstable that it is easily colored upon exposure to oxygen, heat, or light, and that, when Compound A is formulated into pharmaceutical preparations such as injectable compositions, oral dosage forms or suppositories, the coloration is further increased presumably due to the interaction of Compound A with other components such as solvents, diluents or bases.

In addition, Compound A has poor solubility in water (about 4.7 mg/ml at 25°C as free base form). This poor solubility of the compound creates difficulties in the production of injectable compositions of the compound in that the volumes of the injections become too large to be practical, e.g. for intravenous or intramuscular injection and local injection into tumors.

In order to overcome these difficulties, we have made extensive studies, and as a result have found that ionic sulf-oxo compounds containing $HSO_3^-$, $SO_3^{2-}$ or $S_2O_5^{2-}$ ions are effective for the control or prevention of the coloration. As shown in Experimental Example 1 below, various stabilizers were examined in expectation of anticoloration by their antioxidative effects or by chelating of heavy metal ions therewith, but the materials tested proved to have no effect or even to increase said coloration. We have found that ionic sulfoxo compounds containing $HSO_3^-$, $SO_3^{2-}$ or $S_2O_5^{2-}$ ions can effectively prevent the coloration of compound A. When preparations containing compound A and said compounds were tested, an excellent stabilization effect was realised in addition to the anticoloration effect.

The inventors have also found that by the addition to compound A of said compound containing ions selected from $HSO_3^-$, $SO_3^{2-}$ and $S_2O_5^{2-}$ in combination with the secondary additive L-cysteine hydrochloride, it is possible to obtain a stable preparation showing reduced or no coloration even after long storage. Thus an excellent long-term anti-coloration effect was obtained using a combination of sodium metabisulfite with L-cysteine hydrochloride; an injectable composition in an aqueous medium of compound A in combination with sodium metabisulfite and L-cysteine hydrochloride showed no coloration after storing at room temperature for a long period of time or under severe conditions, as shown in Experimental Example 5. This effect was surprising and has not been noted with compositions with other secondary additives. The invention is useful in connection with aqueous injectable compositions which are believed to be the most difficult to stabilize, but can likewise be applied to various other pharmaceutical preparations including oral dosage forms, ointments and suppositories.

Examples of suitable sulf-oxo compounds for use in the invention include bisulfites (e.g. alkali metal bisulfites such as sodium bisulfite ($NaHSO_3$), potassium bisulfite ($KHSO_3$), and ammonium bisulfite ($NH_4HSO_3$)); aqueous sulfurous acid or sulfites (e.g. alkali metal sulfites such as sodium sulfite ($Na_2SO_3$), potassium sulfite ($K_2SO_3$) and alkaline earth metal sulfites such as calcium sulfite ($CaSO_3$), barium sulfite ($BaSO_3$); and metabisulfites (e.g. alkali metal metabisulfite such as potassium metabisulfite ($K_2S_2O_5$) and sodium metabisulfite ($Na_2S_2O_5$)).

From the standpoints of stabilization effect and safety, the preparation according to the invention incorporates from 0.001 to 0.500 part by wt. of said ionic sulf-oxo compound(s) and (when used) from 0.01 to 0.50 part by wt of L-cysteine hydrochloride, per one part by wt. of compound A.

For injection, a suitable daily dose of compound A is 50 to 2000 mg for an adult person.

Studies have also been conducted to find means to improve solubility of the compound A composition. Various surfactants usually employed as solubilizers, additives in anticipation of intramolecular action, and non-aqueous vehicles have been tried, but have failed to give the desired effect. Surprisingly, we have found that appreciable improvement in solubility of compound A composition is obtainable by the addition of basic substance, thus providing for an injection dosage of compound A composition with wide applicability.

The term "basic material" as used herein may include alkali metal hydroxides such as NaOH; alkali metal carbonates such as $Na_2CO_3$; alkaline earth metal hydroxides such as $Ca(OH)_2$; compounds having alkali metal or alkaline earth metal ion as cation, for example borax; and organic amines such as ethanolamine, trishydroxymethyl amino methane, basic amino acids (e.g. L-arginine, L-lysine). Any of the above basic materials can be used, but organic amines are currently preferred in practice.

The solubility of compound A composition tends to increase with the amount of basic material added and with increase in pH. However, for safety, care should be taken over the absolute amount of basic material added, and over selecting the optimum pH range of injection solution to give no local stimulation. The most desirable pH range for an injection solution is 8.0 to 9.5 and under such conditions, the preferable basic materials are organic amines because of ease in handling and quality of the product. The most

2

0 051 962

preferred basic material is L-arginine because of its solubility, safety as an additive and operational ease of handling. If desired, a mixture of basic materials may be used.

The amount of basic material is determined principally by the amount of active ingredient and the overall quality evaluation of the preparation thus obtained taking account of the safety factor, but preferably it is in a range of 0.9 to 3 moles per mole of compound A.

Thus, the present invention makes it possible to provide pharmaceutical preparations of compound A with improved stability and anti-coloration properties by the incorporation of ionic sulf-oxo compound(s) containing at least one of the ions selected from $HSO_3^-$, $SO_3^{2-}$ and $S_2O_5^{2-}$; to attain more complete anticoloration and stabilization effects, even in preparations such as aqueous injectable compositions which are unstable and very liable to discoloration, by the incorporation of L-cysteine hydrochloride in combination with the said sulf-oxo compound(s); and to improve solubility of compound A composition by the incorporation of basic material and especially L-arginine, thereby furnishing a wide variety of injectable compositions of usefully high concentration.

In preparing preparations according to the present invention the above said stabilizer(s) and optionally stabilizer is or are used depending on the requirements of the dosage forms concerned, and other pharmacologically permissible additives such as diluents, bases or solvents can be used as occasion demands for the formulation of various dosage forms following conventional procedures.

Properties and advantages of preparations according to the invention are shown in the following Experimental Examples 1—5:

Experimental Example 1

Each of the stabilizers listed in the following Table 1 was added, to a concentration of 0.2%, to a respective lot of an aqueous solution of compound A (10 mg/ml) containing L-arginine; 5 ml of each resulting solution was placed in a respective 8 ml ampoule, and after replacing the remaining air with nitrogen gas, each ampoule was stored at 50°C for 1 week and the degree of coloration of the contents was then measured by light absorbance at 420 nm (layer thickness 10 mm). Table 1 gives these results and also the colors determined, following JIS Z 8102-1957 "Color Name", Kogyo-yo Shikimeicho, K.K. Nihon Shikisai Sha, by visual inspection.

TABLE 1

| Sample material | Light absorbance | Color |
|---|---|---|
| Control | 0.383 | Pale yellowish green |
| 0.2% Sodium sulfite | 0.038 | Colorless |
| 0.2% Sodium bisulfite | 0.040 | Colorless |
| 0.2% Sodium metabisulfite | 0.035 | Colorless |
| 0.2% EDTA-2Na | 0.520 | Bright yellowish green |
| 0.2% L-ascorbic acid | 0.092 | Faint yellowish green |
| 0.2% Erysorbic acid | 2.786 | Yellow ochre |
| 0.2% Thiourea | 1.016 | Light yellowish green |
| 0.2% Taurine | 0.903 | Light yellowish green |
| 0.2% Acetylthiourea | 0.726 | Light yellowish green |

Experimental Example 2

Freeze-dried preparations containing 20 mg/vial of compound A alone (control) and together with 10 mg/vial of sodium bisulfite were prepared. After 2 months storage at 40°C or 50°C under light shielding conditions or at room temperature under 1000 Lux, the colors were determined by the above visual inspection method and are shown in Table 2:

3

TABLE 2

| | Storage conditions | | |
|---|---|---|---|
| Sample material | Light shielding at 40°C | Light shielding at 50°C | 1000 Lux room temp. |
| Control | White—faint bluish purple | Faint bluish purple | Faint bluish green |
| 10 mg Sodium bisulfite | White | White | White |

Experimental Example 3

Freeze-dried preparations containing 100 mg of compound A and 200 mg of L-arginine alone (control) and together with 5 mg of sodium bisulfite were placed in respective vials under nitrogen gas, and after storing at 50°C under light shielding conditions for 2 months, the contents of compound A were measured from the light absorbance ratios at 276 nm. The results are shown in Table 3.

TABLE 3

| Sample material | % of 4-Carbamoyl-imidazolium-5-oleate originally present |
|---|---|
| Control (without sodium bisulfite) | 89 |
| With 5 mg of sodium bisulfite | 95 |

Experimental Example 4

To respective lots of an aqueous solution of compound A (10 mg/ml) containing L-arginine, various individual and mixed stabilizers were added in an amount of 0.2%; 5 ml of each resulting solution was stored at 50°C for 24 hours in a respective 18 ml stoppered vial and the degrees of coloration were measured by light absorbance at 420 nm (layer thickness 10 nm); the final color was also determined by the above-mentioned visual inspection method. The results, in comparison with those for a corresponding control (no stabilizer) are given in Table 4:

TABLE 4

| Sample material | Light absorbance | Color |
|---|---|---|
| A) Not according to the invention | | |
| Control | 2.392 | Golden |
| 0.2% EDTA-2Na | 1.832 | Golden |
| 0.2% L-ascorbic acid | 1.582 | Dark yellow |
| 0.2% L-cysteine hydrochloride | 1.683 | Golden |
| B) According to the invention | | |
| 0.2% Sodium sulfite | 1.472 | Bright yellow |
| 0.2% Sodium bisulfite | 1.120 | Bright yellow |
| 0.2% Sodium metabisulfite | 1.532 | Bright yellow |
| 0.2% Sodium metabisulfite plus 0.2% EDTA-2Na | 0.833 | Bright yellow |
| 0.2% Sodium metabisulfite plus 0.2% 1-ascorbic acid | 0.601 | Pale yellow |
| 0.2% Sodium metabisulfite plus 0.2% L-cysteine hydro-chloride | 0.286 | Faint yellow |

**0 051 962**

Experimental Example 5

Aqueous injections each containing the following indicated amounts per ampoule were prepared and stored under severe condition of 50°C, 1000 Lux, or at room temperature, and the degrees of coloration were determined by measuring light absorbance at 420 nm (layer thickness 10 mm).

In this example, in preparing the solution, oxygen-free water was used and the free space in the ampoules was filled with nitrogen gas.

Prescription (per ampoule)

| | |
|---|---|
| 4-Carbamoyl-imidazolium-5-olate | 100 mg |
| L-arginine | 200 mg |
| Sodium metabisulfite | 4 mg |
| L-cysteine hydrochloride | 10 mg |
| Benzyl alcohol | 50 mg |
| Sterilized distilled water for injection | to make 5 ml |

TABLE 5

| Storage condition | Light absorbance |
|---|---|
| Initial | 0.020 |
| 50°C—2 months | 0.002 |
| 1000 Lux—2 months | 0.007 |
| Room temp.—6 months | 0.015 |

Reference Example 1 (not according to the invention)

Excess amounts of Compound A were dispersed in aqueous solutions containing various surfactants and additives, respectively. In each case, the solubility of the compound was determined by measuring light absorbance of the filtrate at 277 nm. For non-aqueous vehicles marked with *, the corresponding solubility of the compound was judged by eye-measurement.

5

### TABLE 6

| Solvent | Solubility mg/ml of 4-carbamoyl-imidazolium-5-olate | Solvent | Solubility mg/ml of 4-carbamoyl-imidazolium-5-olate |
|---|---|---|---|
| Water | 4.7 | 5% Amycol No. 1 (manufactured by Nichiden Kagaku K.K.) | 5.1 |
| 5% Polyoxyethylene hydrogenated castor oil | 4.5 | 10% Sodium deoxycholate | White gel |
| 5% Polyoxyethylene sorbitan mono-olate | 4.6 | Saturated glutamic acid | 5.3 |
| 5% Sodium lauryl sulfate | 4.7 | Saturated aspartic acid | 5.4 |
| * Ethanol | less than 2 | Saturated gentisic acid | White gel |
| * Propylene glycol | about 5 | 5% Levulinic acid | 6.3 |
| * Polyethylene glycol | less than 2 | 5% Tartaric acid | 7.0 |
| * Glycerine | about 3 | 10% Mannitol | 4.6 |
| * N,N-dimethyl-acetamide | less than 2 | 5% Sodium salicylate | 6.5 |
| * Sesame oil | less than 2 | 5% Nicotinic amide | 7.2 |
| * Cotton seed oil | less than 2 | 5% Ethylurea | 5.6 |
| * Corn oil | less than 2 | | |

Reference Example 2 (not according to the invention)

Using the same procedures as in Reference Example 1, further solubility measurements were made with the following results:

### TABLE 7

| Solvent | Solubility of 4-carbamoyl-imidazolium-5-olate, mg/ml | pH of solvent |
|---|---|---|
| 1% Ethanolamine | 24.9 | 8.64 |
| 5% Trishydroxy methyl-amino methane | 25.7 | 8.56 |
| 5% L-arginine | 33.5 | 8.69 |
| 5% L-lysine | 10.7 | 8.20 |
| 0.5% NaOH | 21.4 | 8.50 |
| $NaHCO_3$-$Na_2CO_3$ buffer | 26.7 | 8.42 |
| 5% Borax | 22.2 | 8.44 |

The invention is more fully illustrated by the following Examples.

Example 1

A mixture of 4-carbamoyl-imidazolium-5-olate (100 g), sodium bisulfite (5 g) and L-arginine (200 g) was dissolved in distilled water for injection to give a total volume of 10 l. The solution was filtered to remove bacteria, and 10 ml lots were poured into vials (24 ml in volume) and freeze-dried (the free vial space being filled with nitrogen gas) to obtain freeze-dried compositions; these were stable and capable of being reconstituted for use in high concentration in less than 10 ml of distilled water for injection.

Example 2

The same procedures as in Example 1 were repeated, except for substituting sodium sulfite for sodium bisulfite, to obtain freeze-dried compositions which were stable and capable of being reconstituted in high concentration in less than 10 ml of distilled water for injection.

Example 3

The same procedures as in Example 1 were repeated, except for substituting sodium metabisulfite for sodium bisulfite, to obtain freeze-dried compositions which were stable and capable of being reconstituted in high concentration in less than 10 ml of distilled water for injection.

Example 4

A mixture of 4-carbamoyl-imidazolium-5-olate (100 g), sodium bisulfite (2.5 g), sodium metabisulfite (2.5 g) and L-arginine (200 g) was dissolved in distilled water for injection to give a total volume 10 l.

The solution was filtered to remove bacteria, and 10 ml lots were poured into vials (24 ml in volume) and subjected to freeze-drying to obtain freeze-dried compositions; these were stable and capable of being reconstituted in high concentration in less than 10 ml of distilled water for injection.

Example 5

156 g of 4-carbamoyl-imidazolium-5-olate hydrochloride, 3.1 l of 1% aqueous sodium hydroxide solution and 200 g of L-arginine were dissolved with stirring, in distilled water for injection, to make a total volume of 10 liters. This was filtered to remove bacteria, and 10 ml lots were poured into vials (24 ml in volume) and subjected to freeze-drying to obtain freeze-dried compositions; these were stable and capable of being reconstituted in high concentration in less than 10 ml of distilled water for injection.

Example 6

Into 5 liters of oxygen-free, sterilized, distilled water for injection, were dissolved gently 4 g of sodium metabisulfite, 10 g of L-cysteine hydrochloride and then 100 g of 4-carbamoyl-imidazolium-5-olate, 200 g of L-arginine and 50 g of benzyl alcohol. The resulting solution was filtered under sterile conditions, each 5 ml lots were then filled into ampoules, and after replacing air in the ampoules with nitrogen gas, the ampoules were sealed to obtain aqueous compositions of 4-carbamoyl-imidazolium-5-oleate which were stable for a long period.

Example 7

| | |
|---|---|
| Compound A | 2000 g |
| Crystalline cellulose | 2400 g |
| Magnesium stearate | 50 g |
| Sodium bisulfite | 150 g |

The above-mentioned ingredients were mixed together and the resulting powdery mixture was formed into tablets each weighing about 230 mg. These proved to be very stable for a long period.

Example 8

| | |
|---|---|
| Compound A | 400 g |
| Milk sugar | 600 g |
| Sodium metabisulfite | 20 g |
| L-cysteine hydrochloride | 40 g |

The above-mentioned ingredients were mixed together to obtain a uniformly mixed powder, which was stable for a long period.

**Claims**

1. A pharmaceutical preparation containing (a) an active ingredient selected from 4-carbamoyl-

imidazolium-5-olate and salts and hydrates thereof, and (b) stabilizer, characterised in that the stabilizer comprises at least one ionic sulf-oxo compound containing at least one of the ions $HSO_3^-$, $SO_3^{2-}$ and $S_2O_5^{2-}$, said ionic sulf-oxo compound(s) amounting to from 0.1 to 50% by weight of (a).

2. A preparation according to claim 1 characterised in that the stabiliser also includes L-cysteine hydrochloride.

3. A pharmaceutical preparation according to claim 1 or 2, further characterised in that it also contains a basic substance as a solubiliser.

4. A composition according to claim 3 characterised in that said basic substance comprises an organic amine, preferably L-arginine.

5. An injectable composition according to any of claims 3 or 4.

6. A method of stabilising, and/or inhibiting discoloration of, a pharmaceutical preparation of (a) an active ingredient selected from 4-carbamoyl-imidazolium-5-olate and salts and hydrates thereof by incorporating (b) stabilizer therein, characterised in that the stabilizer comprises (c) at least one ionic sulf-oxo compound containing at least one of the ions $HSO_3^-$, $SO_3^{2-}$ and $S_2O_5^{2-}$ and optionally (d) L-cysteine hydrochloride, said ionic sulf-oxo compound(s) amounting to from 0.1 to 50% by weight of (a).

7. A method of enhancing water solubility of a preparation according to claim 1 or 2, characterised by mixing it with a basic substance.

8. A method of producing an injectable composition of a preparation according to claim 1 or 2 characterised by dissolving the preparation in combination with a basic substance in an aqueous medium.

9. A method according to claim 7 or 8 characterised in that said basic substance comprises an organic amine, preferably L-arginine.

**Patentansprüche**

1. Pharmazeutisches Mittel, enthaltend (a) einen Wirkstoff ausgewählt aus 4-Carbamoyl-imidazolium-5-olat und Salzen sowie Hydraten davon, und (b) einen Stabilisator, dadurch gekennzeichnet, daß der Stabilisator wenigstens eine ionische Sulfoxoverbindung aufweist, die wenigstens eines der Ionen $HSO_3^-$, $SO_3^{2-}$ und $S_2O_5^{2-}$ enthält, wobei die ionische Sulfoxoverbindung(en) 0,1 bis 50 Gew.-% von (a) ausmacht (ausmachen).

2. Mittel nach Anspruch 1, dadurch gekennzeichnet, daß der Stabilisator auch L-Cysteinhydrochlorid enthält.

3. Pharmazeutisches Mittel nach Anspruch 1 oder 2, dadurch gekennzeichnet, daß es auch eine basische Substanz als Solubilisierungsmittel enthält.

4. Mittel nach Anspruch 3, dadurch gekennzeichnet, daß die basische Substanz aus einem organischen Amin, vorzugsweise L-Arginin, besteht.

5. Injizierbares Mittel nach einem der Ansprüche 3 oder 4.

6. Verfahren zum Stabilisieren und/oder zum Hemmen einer Verfärbung einer pharmazeutischen Zubereitung aus (a) einem Wirkstoff, ausgewählt aus 4-Carbamoylimidazolium-5-olat sowie Salzen und Hydraten davon, durch Einmengen von (b) eines Stabilisierungsmittels, dadurch gekennzeichnet, daß das Stabilisierungsmittel (c) wenigstens eine ionische Sulfoxoverbindung, die wenigstens eines der Ionen $HSO_3^-$, $SO_3^{2-}$ und $S_2O_5^{2-}$ enthält, sowie gegebenenfalls (d) L-Cysteinhydrochlorid aufweisen, wobei die ionische Sulfoxoverbindung(en) 0,1 bis 50 Gew.-% von (a) ausmacht (ausmachen).

7. Verfahren zur Erhöhung der Wasserlöslichkeit eines Mittels gemäß Anspruch 1 oder 2, dadurch gekennzeichnet, daß es mit einer basischen Substanz vermischt wird.

8. Verfahren zur Herstellung einer injizierbaren Zubereitung eines Mittels gemäß Anspruch 1 oder 2, dadurch gekennzeichnet, daß das Mittel in Kombination mit einer basischen Substanz in einem wäßrigen Medium aufgelöst wird.

9. Verfahren nach Anspruch 7 oder 8, dadurch gekennzeichnet, daß die basische Substanz aus einem organischen Amin, vorzugsweise L-Argining, besteht.

**Revendications**

1. Préparation pharmaceutique contenant (a) un ingrédient actif choisi entre le 4-carbamoyl-imidazolium-5-olate et ses sels et hydrates, et (b) un stabilisant, caractérisée en ce que le stabilisant comprend au moins un composé renfermant un groupement ionique sulfoxo, contenant au moins l'un des ions $HSO_3^-$, $SO_3^{2-}$ et $S_2O_5^{2-}$, le ou les composés renfermant un groupement ionique sulfoxo étant présents en une quantité de 0,1 à 50% en poids de (a).

2. Préparation suivant la revendication 1, caractérisé en ce que le stabilisant comprend également du chlorhydrate de L-cystéine.

3. Préparation pharmaceutique suivant la revendication 1 ou 2, caractérisée en outre en ce qu'elle contient également une substance basique servant de solubilisant.

4. Composition suivant la revendication 3, caractérisée en ce que la substance basique comprend une amine organique, de préférence la L-arginine.

5. Composition injectable suivant la revendication 3 ou 4.

6. Procédé pour stabiliser et/ou empêcher la décoloration d'une préparation pharmaceutique comprenant (a) un ingrédient actif choisi entre le 4-carbamoylimidazolium-5-olate et ses sels et hydrates auxquels est incorporé (b) un stabilisant, caractérisé en ce que le stabilisant comprend (c) au moins un composé renfermant un groupement ionique sulfoxo, contenant au moins l'un des ions $HSO_3^-$, $SO_3^{2-}$ et $S_2O_5^{2-}$, et facultativement, (d) du chlorhydrate de L-cystéine, le ou les composés renfermant un groupement ionique sulfoxo étant présents en une quantité de 0,1 à 50% en poids de (a).

7. Procédé pour accroître l'hydrosolubilité d'une préparation suivant la revendication 1 ou 2, caractérisé en ce qu'il consiste à mélanger celle-ci à une substance basique.

8. Procédé de production d'une composition injectable renfermant une préparation suivant la revendication 1 ou 2, caractérisé en ce qu'il consiste à dissoudre la préparation associée à une substance basique dans un milieu aqueux.

9. Procédé suivant la revendication 7 ou 8, caractérisé en ce que la substance basique comprend une amine organique, de préférence la L-arginine.